# EUROPÄISCHE PATENTSCHRIFT

(11) **EP 1 897 485 B1**
(45) Veröffentlichungstag und Bekanntmachung des Hinweises auf die Patenterteilung: **21.09.2011**
(21) Anmeldenummer: 06018878.6
(22) Anmeldetag: 08.09.2006
(51) Int. Cl.: A61B 5/00, G06F 19/00

(54) **Verfahren zur Analyse und/oder Überwachung physiologischer und/oder pathologischer Parameter eines Patienten**
Method for analysing and/or monitoring physiological and/or pathological parameters of a patient
Procédé d'analyse et/ou de surveillance de paramètres physiologiques et/ou pathologiques d'un patient

(43) Veröffentlichungstag der Anmeldung: 12.03.2008
(73) Patentinhaber: Löser Medizintechnik GmbH, 04275 Leipzig (DE)
(72) Erfinder: Löser, Thomas, 04275 Leipzig (DE)
(74) Vertreter: Carlsohn, Alexander

(56) Entgegenhaltungen:
- WO-A1-00/05671
- WO-A2-03/020128

## Beschreibung

Die Erfindung betrifft ein Verfahren zur Analyse und/oder Überwachung physiologischer und/oder pathologischer Parameter eines Patienten. Das Verfahren ist insbesondere zur Früherkennung von Krankheitszuständen und zur Generierung von Handlungsvorschlägen zur Vermeidung von Krankheitszuständen geeignet, die multiparametrisch zu diagnostizieren sind, wie z. B. die Bildung von Metastasen, die Entwicklung einer beatmungsassoziierten Pneumonie, die Herausbildung eines schnell verlaufenden Knochenmasseverlustes oder die Entstehung verschiedener Organausfälle in der Folge einer Sepsis.

Die Frühdiagnostik von verschiedenen Krankheitszuständen und die Einleitung einer evidenzbasierten Therapie in der Frühphase der Erkrankung ist nach wie vor ein ungelöstes Problem. Medizinische Diagnosen und Therapieentscheidungen werden in vielen Fällen anhand von Meßwerten physiologischer und/oder pathologischer Parameter getroffen. Derartige Meßwerte umfassen beispielsweise Meßwerte für Indikatorstoffe, deren Vorhandensein und/oder deren Quantität in einer Probe, wie beispielsweise Körperflüssigkeit, Rückschlüsse auf einen bestimmten Krankheitszustand zulassen sollen. Zur Bewertung eines Meßwertes wird in der Regel ein Klassifikationsverfahren verwendet, bei dem zwischen zwei Klassen (z. B. Klasse A: negativer Befund; Klasse B: positiver Befund) unterschieden wird.

Ein weiteres, noch schwierigeres Problem ist die Ableitung von Handlungsweisen in der Frühtherapie, d. h. welche Maßnahme ist geeignet, ein künftiges wahrscheinliches Ereignis zu verhindern bzw. seine Ereignisschwere zu reduzieren.

Die Einordnung eines Meßergebnisses in die jeweilige Klasse erfolgt anhand eines Schwellwertes. Liegt der erhaltene Meßwert über dem Schwellwert (erhöhter Wert) und sind hohe Werte typischerweise mit dem Erkrankungsbild verbunden, läßt das auf eine Erkrankung schließen, liegt er unterhalb dieses Schwellwertes, kann nicht auf eine Erkrankung geschlossen werden.

Diese Verfahrensweise zur Diagnose von Krankheiten ist ein einparametrisches Klassifikationsverfahren, das sich lediglich auf die Zuordnung des Meßwertes zu einer von zwei Klassen beschränkt. Unberücksichtigt bleibt dabei allerdings, wie weit der Meßwert von dem jeweiligen Schwellwert entfernt ist. Das hat zur Folge, das ein Meßwert knapp unterhalb des Schwellwertes ebenso in dieselbe Klasse eingeordnet wird wie ein Meßwert, der sehr weit unterhalb des Schwellwertes liegt.

In der Anwendung eines Klassifikators mit quantitativer Ausgangsgröße wird die Sicherheit des Ergebnisses durch zwei Charakterisierungen umschrieben: Zum einen zeigt die Sensitivität/Spezifität des verwendeten Klassifikators die Leistungsfähigkeit des Verfahrens, zum anderen gibt der Abstand des Ausgangswertes zum Schwellwert die Trennfähigkeit für den konkreten Datensatz an. Diese herkömmliche Verfahrensweise zur Diagnose einer Erkrankung anhand von Indikatorstoffen vermittelt zwar einen Anhaltspunkt, hat aber schwerwiegende Nachteile: Es erfordert vom Anwender ein tiefgreifendes Systemverständnis, um die Ergebnissicherheit im Hinblick auf eine zutreffende Diagnose zu bewerten. Darüber hinaus kann die Entscheidungssicherheit nur bezüglich der Trennschwelle bewertet werden, der quantitative Vergleich zweier Ergebnisse hinsichtlich ihrer Aussagesicherheit ist nicht möglich. Dies gilt erst bei der Auswertung mehrerer Meßwerte. Erhält man für zwei Meßwert-Datensätze den gleichen (mehrdimensionalen) Abstand zum Schwellwert, so sagt dies nichts über die qualitative Vergleichbarkeit der zugrundeliegenden Fälle aus (sie können sich in ihrer Fallschwere durchaus deutlich unterscheiden).

In der Regel ist es zur Diagnose einer Erkrankung, beispielsweise eines Lungenkarzinoms, nicht ausreichend, lediglich einen Indikatorstoff zu verwenden. Werden jedoch mehrere Indikatorstoffe verwendet und jeweils einzeln bewertet, werden oft widersprüchliche Ergebnisse erhalten, da für jeden Indikatorstoff eine gesonderte Klassifizierung in die Negativ- oder Positiv-Klasse vorgenommen wird, wobei jede Klassifizierung mit einer speziellen Spezifität und Sensitivität verbunden ist.

Die Bewertung von Sensitivität oder Spezifität von Meßwerten physiologischer Parameter läßt jedoch das Zusammenwirken einer Vielzahl von Parametern in einem Individuum weitgehend unberücksichtigt. Ferner sind die Klassifikationsverfahren in der Regel zur Feststellung, ob eine Erkrankung vorliegt geeignet, nicht aber für die Früherkennung von Erkrankungen, beispielsweise die Assoziation von Tumorzellen zu Tumoren.

WO 00/05671 A1 offenbart ein Verfahren zur Analyse der Entwicklung eines biologischen Systems. Dieses Verfahren umfaßt das Bestimmen einer Reihe von Variablen, von denen der Zustand des biologischen Systems abhängt, wobei die Variabeln in einem mehrdimensionalen Raum abgebildet werden. Die Entwicklung wird nun unter Verwendung einer Trajektorie überwacht, die aus Gruppen der Variabeln gebildet wird, wobei die Gruppen den Zustand des Systems zu unterschiedlichen Zeitpunkten definieren. Dabei wird die Zeit als ein Parameter des mehrdimensionalen Raumes so verwendet, daß jeder Punkt auf der Trajektorie zumindest einem Zeitwert entspricht.

Aufgabe der Erfindung ist es, die Nachteile nach dem Stand der Technik zu beseitigen. Es soll insbesondere ein Verfahren angegeben werden, mit dem die Früherkennung von Krankheiten auf der Basis physiologischer und pathologischer Parameter des Patienten ermöglicht wird und zu jedem Zeitpunkt der Krankheitsentwicklung oder des Genesungsprozesses einen Handlungswert ausgegeben werden kann, der geeignet ist, den Patientenzustand aus dem gefährdeten Bereich in den Zielbereich zu therapieren.

Diese Aufgabe wird durch die Merkmale des Anspruchs 1 gelöst. Zweckmäßige Ausgestaltungen der Erfindungen ergeben sich aus den Merkmalen der Ansprüche 2 bis 12.

Die Anzahl der ausgewählten Parameter beträgt vorzugsweise mindestens 5, besonders bevorzugt mindestens 10.

Der erste Meßpunkt sowie die weiteren Meßpunkte bilden eine Raumkurve in dem Merkmalsraum, die nachfolgend auch als Patiententrajektorie bezeichnet wird.

Die kürzesten Raumkurven zwischen dem ersten Zustand und einem zweiten Raumbereich wird als Komplikationstrajektorie bezeichnet. Liegt einer der weiteren Meßpunkte auf der Komplikationstrajektorie oder entwickelt sich die Patiententrajektorie in Richtung der Komplikationstrajektorie so ist eine Behandlung des Patienten dringend erforderlich. Auf dieses Dringlichkeit kann mittels des Handlungswertes hingewiesen werden.

Der erfindungsgemäß vorgesehene Merkmalsraum, der zumindest einen ersten und einen zweiten Raumbereich aufweist, ermöglicht die frühzeitige Erkennung, ob die Entwicklung des Patienten in Richtung des erwünschten Zustandes (erster Raumbereich) oder in Richtung des unerwünschten Zustandes (zweiter Raumbereich) erfolgt. Erfolgt die Entwicklung des Zustandes des Patienten nicht entlang der Zieltrajektorie, so ist dies frühzeitig feststellbar. Es kann ferner ermittelt werden, welcher Parameter verändert werden muß, um eine Entwicklung der Patiententrajektorie auf kürzestem Wege in Richtung des ersten Raumbereiches oder der Zieltrajektorie zu erreichen. Dieser Parameter wird hier auch als Handlungswert bezeichnet, wobei der Handlungswert auch mehrere Parameter zusammenfassen kann, die verändert werden müssen, um eine Entwicklung der Patiententrajektorie auf kürzestem Wege in Richtung des ersten Raumbereiches oder der Zieltrajektorie zu erreichen.

Der Handlungswert besteht in der Angabe zumindest eines Parameters, der verändert werden muß, um die Patiententrajektorie auf kürzestem Wege in Richtung des ersten Raumbereiches oder der Zieltrajektorie zu führen. Aus dieser Angabe kann ein Behandler entnehmen, welche Maßnahmen erforderlich sein, um den angegebenen Parameter zu verändern, beispielsweise durch Gabe eines pharmazeutischen Wirkstoffes.

Auf Basis dieser Erkenntnis können zur Früherkennung von Erkrankungen wie beispielsweise Tumoren nunmehr Merkmalsräume gebildet werden, die eine Früherkennung von physiologischen Zuständen ermöglichen, bei denen eine wirksame Tumorabwehr nicht mehr möglich ist.

Die nachfolgende Tabelle 1 zeigt beispielhaft Parameter für den Ausgangzustand, einen einzigen ersten Raumbereich und zwei zweite Raumbereiche.

**Tabelle 1**

| Parameter | Ausgangszustand | Erster Raumbereich | Zweiter Raumbereich 1 | Zweiter Raumbereich 2 |
|---|---|---|---|---|
| 1 | + | + | - | - - |
| 2 | - | + | + | - - |
| 3 | - | + | - - - | - |
| 4 | - | + | - - | - |
| 5 | - - | + | - | - - |
| 6 | - | + | - - - | - |
| 7 | - - - | + | - | - - |
| 8 | - | + | + | - |
| 9 | - | + | + | - |
| 10 | - - | + | - | - - |
| 11 | - - - | + | - - - | - - - |
| 12 | - | + | - - - | + |

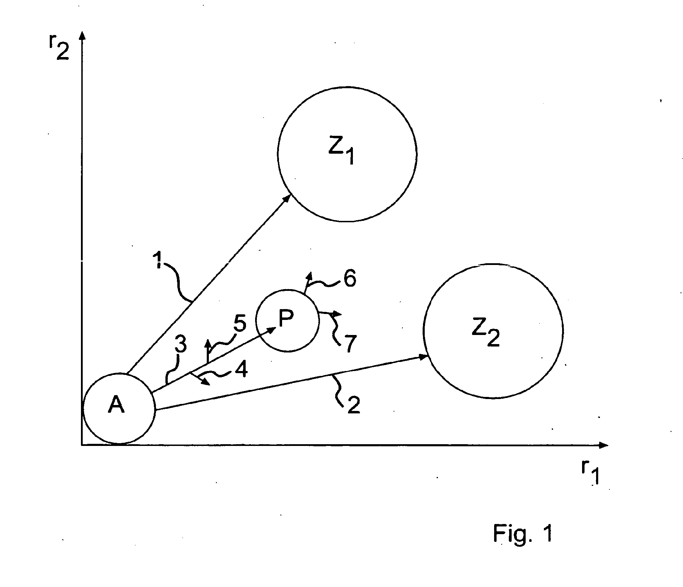

Die Tabelle zeigt 12 Parameter, so daß der Merkmalsraum 13 Dimensionen aufweist (12 Parameter sowie die Zeit). Für den Ausgangszustand sind die Meßwerte für die Parameter beispielhaft mit "schlecht" (-), "sehr schlecht" (- -) und "besonders schlecht"(- - -) wiedergegeben. Der erwünschte Zustand, d. h. der erste Raumbereich, ist so definiert, daß die Meßwerte für alle Parameter einen Wert erreicht haben, der als "gut" (+) zu bewerten ist. In den zweiten Raumbereichen 1 und 2 sind die Werte für die Parameter jedoch nicht durchgehend gut, sondern teilweise "schlecht", "sehr schlecht" oder "besonders schlecht". Die Entwicklung des Patienten, die durch die Patiententrajektorie wiedergeben wird, soll die zweiten Raumbereiche nicht erreichen. Aus diesem Grunde ist erfindungsgemäß vorgesehen, daß bei Abweichungen der Patiententrajektorie, d. h. der Raumkurve zwischen dem Ausgangszustand A und dem gegenwärtigen Zustand des Patienten P (siehe Fig. 1), von der Zieltrajektorie, d. h. der Raumkurve zwischen dem Ausgangszustand A und dem ersten Bereich Z₁, ein Handlungswert bestimmt wird, der eine Rückführung der Patiententrajektorie zu der Zieltrajektorie oder dem ersten Raumbereich Z₁ ermöglicht.

Bei der Auswahl der Parameter, die zur Bildung des Merkmalsraums herangezogen, werden die physiologischen und/oder pathologischen Parameter bevorzugt, die für den Zustand des Gewebes des Patienten, das Alter des Patienten und die Zusammensetzung des Gewebes charakteristisch sind. Besonders bevorzugte Parameter, die für die Zusammensetzung des Gewebes charakteristisch sind, sind die stoffliche und/oder die zelluläre Zusammensetzung des Gewebes.

Bevorzugte Parameter sind aus der Gruppe ausgewählt, die
- Parameter, die für akute oder frühere Entzündungen des Patienten charakteristisch sind;
- Parameter, die für den Ernährungszustand des Patienten charakteristisch sind;
- Parameter, die für akute oder frühere Infektionen des Patienten charakteristisch sind; und
- Parameter, die für den physiologische Alterungsprozeß charakteristisch sind;
- Parameter, die für den Wasser- und Elektrolytstoffwechsel und die Mineralstoff- und Spurenelementversorgung charakteristisch sind;
- Parameter, die für die Hormonlage charakteristisch sind;
- Parameter, die für den Bindegewebsstoffwechsel charakteristisch sind;
- Parameter, die für die Hämostase charakteristisch sind;
- Parameter, die für die Durchblutung charakteristisch sind
- Parameter, die für die Immunüberwachung charakteristisch sind;
- Parameter, die für Diabetes charakteristisch sind; und
- Parameter, die für Therapie charakteristisch sind, wie z. B. Parameter für Bestrahlungs- und Medikamentendosen; umfaßt.

Bevorzugte Parameter, die für den Ernährungszustand des Patienten charakteristisch sind, sind Parameter, die für den Wasser- und Elektrolytstoffwechsel des Patienten charakteristisch sind, und/oder Parameter, die für die Spurenelementversorgung charakteristisch sind.

Bevorzugte Parameter, die für den physiologischen Alterungsprozeß charakteristisch sind, sind die Konzentration vorgegebener Hormone, Parameter, die für Veränderungen des Bindegewebsstoffwechsels charakteristisch sind, und Parameter, die mit Diabetes assoziierte Parameter umfassen.

Die Parameter können kontinuierlich oder diskontinuierlich erfaßt werden. Der erzeugte Handlungswert kann an ein herkömmliches Patientenwarnsystem übergeben werden, daß dann beispielsweise einen optischen oder akustischen Alarm auslöst. Ebenso ist eine direkte Auslösung eines Warnsignals möglich.

Der Merkmalsraum ist ein mehrdimensionaler Raum, wobei eine der Dimensionen die Zeit ist.

Beispielhafte Parameter zur Beurteilung des Risikos einer Tumorbildung sind im Falle eines N.N.-Tumors folgende Parameter: kurativ behandeltes Mammakarzinom, Genotyp A, Osteoporose, chronische Raucherin, Dehydration, akute Entzündung, schlechter Mineralstoff- und Spurenelementstatus.

Mindestens zwei der in Schritt (a) ausgewählten physiologischen und/oder pathologischen Parameter sind chemische Indikatorstoffe, die für das Vorliegen einer Erkrankung charakteristisch sind.

Der Begriff "Indikatorstoff" bezieht sich hierin auf Verbindungen oder Elemente, die - je nach ihrer Art - in biologischen Systemen produziert werden oder in biologischen Systemen eingebracht werden und deren Vorhandensein oder deren Konzentration (z. B. in einem bestimmten Organ) ein Charakteristikum für einen biologischen Prozeß oder einen biologischen Zustand ist. Derartige Verbindungen und Elemente umfassen beispielsweise solche, die von Tumorzellen produziert, durch einen Tumor in anderen Körperzellen induziert und/oder als tumorspezifische Stoffe in ihrer Konzentration durch einen Tumor verändert werden. Derartige Indikatorstoffe sind beispielsweise Makromoleküle, z. B. Proteine, oder Spurenelemente. Derartige Verbindungen und Elemente umfassen weiterhin Bonemarker, die für Knochenabbauprozesse wie Osteoporose charakteristisch sind.

Bei der Auswahl der Parameter in Schritt (a) werden vorzugsweise auch Parameter ausgewählt, die für den Genotyp des Patienten charakteristisch sind. Basierend auf den Hauptgenotypen können zweite Raumbereiche definiert und Komplikationstrajektorien bestimmt werden, von denen der erste Raumbereich und damit die Zieltrajektorie abgegrenzt werden kann.

### Die Erfindung beruht auf folgenden Erkenntnissen:

Der Organismus macht von seiner ersten Entstehung bei der Befruchtung bis zum Tod, gegenüber der Zeit aufgetragen, eine Reihe von Zustandsänderungen durch. Die Zustandsänderungen können mit Hilfe von Meßgrößen wie Größe, Gewicht, Labor- und Funktionsparameter, Bildern u. a. verfolgt werden. Die Meßgrößen spannen einen Merkmalsraum bzw. Phasenraum auf, wo der Organismus zu jedem Zeitpunkt seiner Existenz einen anderen Ort einnimmt. Er beschreibt eine Raumkurve über der Zeit, eine Trajektorie. Die Idealtrajektorie ist eine ungestörte Trajektorie, die allein durch die physiologischen Veränderungen des Organismus über der Zeit wie z. B. Knochenmasseaufbau und Knochenmasseverlust, die hormonellen Umstellungen von der Pubertät zur Menopause, die Abnahme von Grundumsatz, Herzindex, Vitalkapazität, Muskelkraft, des Bindegewebes u.v.a.m. bestimmt ist.

Der Merkmalsraum kann als universell, d. h. für jeden Vertreter einer Spezies als gültig angesehen werden. Durch den jeweils unterschiedlichen Genotyp ergibt sich jedoch eine starke Individualisierung in den Ausgangsbedingungen bzw. dem Startpunkt der individuellen Trajektorie. Der Startpunkt der individuellen Trajektorie kann deren Verhalten nach einer Störung stark beeinflussen, d. h. an sich gleichartige Trajektorien, jedoch mit unterschiedlicher Startbedingung, können im Fall 1 ein stabiles Verhalten besitzen und im Fall 2 eine sich aufschaukelnde Schwingung zur Folge haben.

Die allgemeine Lösung der Lebenstrajektorie ist teilweise beschrieben und von Interesse für die unterschiedlichsten Fragestellungen, die Ernährungswissenschaften, die Geriatrie u.a.

Für akute medizinische Fragestellungen sind Teilstücke der allgemeinen Lebenstrajektorie von Bedeutung, z.B. das Verhalten eines Organs oder des Organismus vor bzw. nach definierten Erkrankungen oder Eingriffen. Für diese Trajektorienteilstücke kann ein gegenüber der allgemeinen Lösung reduzierter Merkmalsraum verwendet werden, der wiederum über der Zeit abgebildet wird. Das betreffende Organ bzw. der Organismus legt in dem betrachteten Zeitfenster eine Bewegung im Merkmalsraum zurück.

Der Anfangszustand wird durch ein definiertes Ereignis markiert, z. B. den Abschluß einer Operation, den Beginn der Beatmung, der Diagnose einer Sepsis oder viele andere mehr. Aus dem Anfangszustand können sich verschiedene Endzustände entwickeln, z. B. nach einer erfolgreichen kurativen Mammakarzinom-Operation innerhalb von 20 Jahren keine Metastasen entwickeln, ein anderer Endzustand ist die Entwicklung einer Metastase oder eine postoperative Komplikation, z.B. eine Pneumonie oder eine Sepsis. In allen Fällen beschreibt der Organismus im Beobachtungszeitraum jeweils eine unterschiedliche Bahnkurve innerhalb des Merkmalsraums. Die Trajektorie für das erwünschte Ereignis wird als Zieltrajektorie, alle anderen Trajektorien als Komplikationstrajektorien bezeichnet.

Durch Aufnahme der Trajektorien für ein erzieltes Ereignis, z. B. für das metastasenfreie Überleben von 20 Jahren nach einer Mammakarzinom-Operation und für verschiedene unerwünschte Ereignisse, z. B. Metastasen nach 5 Jahren oder eine Sepsis innerhalb von 2 Wochen postoperativ kann man nun steuernd tätig werden.

Voraussetzung für die Steuerung ist eine häufige Erfassung der definierten Beobachtungsparameter des jeweiligen Patienten. Aus zwei Parametersätzen kann bereits ein Trajektorienteilstück errechnet werden. Die Lage der individuellen Patiententrajektorie gegenüber der bereits bekannten Zieltrajektorie und den verschiedenen Komplikationstrajektorien wird bestimmt. Liegt die Patiententrajektorie innerhalb des Toleranzbandes der Zieltrajektorie ist keine zusätzliche Handlung erforderlich, liegt sie außerhalb und in Nähe einer der Komplikationstrajektorien, so wird der kürzeste Weg in Richtung der Zieltrajektorie bestimmt und als Handlungswert ausgegeben. Weichen mehrere Beobachtungsparameter ab, so wird eine Wichtung der Handlungsparameter vorgenommen. Das kann an unterschiedlichen Bahnpunkten der Trajektorie, d. h. zu unterschiedlichen Zeiten innerhalb des Erkrankungs- bzw. Genesungsverlaufs eine unterschiedliche Handlungsgröße sein.

Der Merkmalsraum und die Raumbereiche werden anhand der ausgewählten Parameter wie folgt gebildet:
(a) Bestimmen der physiologischen und/oder pathologischen Parameter, die für eine Erkrankung, beispielsweise eine Tumorbildung in einem vorgegebenen Tumorgewebe, charakteristisch sind.
(b) Bilden des Merkmalsraumes, wobei jeder der Parameter eine Dimension des Merkmalsraumes bildet und die Zeit eine zusätzliche Dimension des Merkmalsraumes ist.
(c) Bereitstellen von Meßwerten dieser physiologischen Parameter aus Patientendatenbanken. Die Meßwerte können beispielsweise in klinischen Studien gewonnen werden. Die Meßwerte sind vollständig oder teilweise mit Beschreibungen wie "krankhaft" oder "gesund" gekennzeichnet. Vorzugsweise sind die Meßwerte eines Parameters mit Meßwerten eines oder mehrerer anderer Parameter verknüpft, wobei die Verknüpfungen vollständig oder teilweise mit Beschreibungen wie "krankhaft" oder "gesund" gekennzeichnet sind
(d) Definieren der ersten und zweiten Raumbereiche in dem Merkmalsraum, wobei der erste Raumbereich anhand der Meßwerte oder Verknüpfungen gebildet wird, die als "krankhaft" beschrieben sind, während der zweite Raumbereich anhand der Meßwerte oder Verknüpfungen gebildet wird, die als "gesund" beschrieben werden.

Die Erfindung wird nachfolgend anhand von Beispielen sowie der Zeichnung näher erläutert. Dabei zeigt
- Fig. 1: ein Diagramm, daß Merkmalsräume sowie erste und zweite Bereiche in bezug auf Raumkoordinaten zeigt.

Das in Fig. 1 gezeigte Diagramm zeigt einen mehrdimensionalen Merkmalsraum, wobei nur zwei Dimensionen dargestellt sind. Die Koordinaten r₁ und r₂ stellen zwei der ausgewählten Parameter dar. Parameter r₁ ist beispielsweise die Zeit.

Kreis A beschreibt schematisch den Ausgangszustand eines Patienten, beispielsweise nach einer Tumoroperation, anhand der ausgewählten Parameter. Dazu werden die Meßwerte der Parameter in den Merkmalsraum eingetragen. Der Zustand des Patienten kann sich nun so entwickeln, daß er direkt den ersten Raumbereich Z₁ (Pfeil 1) oder den zweiten Raumbereich Z₂ (Pfeil 2) erreicht. Die Entwicklung in Richtung Pfeil 1 ist erwünscht und wird als Zieltrajektorie bezeichnet. Die Entwicklung in Richtung Pfeil 2 ist unerwünscht und wird als Komplikationstrajektorie bezeichnet.

Die tatsächliche Entwicklung dieser Parameter im Verlauf der Zeit (Pfeil 3, Patiententrajektorie) wird verfolgt, wobei der Patient in den Zustand P gelangt. Änderungen der Entwicklungsrichtungen sind jedoch möglich (Pfeile 4 und 5). Die Entwicklung des Zustandes des Patienten entlang Pfeil 3 ist erkennbar nicht optimal, da der erstrebte Zustand Z₁ bei Fortsetzung der Entwicklung in dieser Richtung nicht erreicht werden kann. Es ist somit eine medizinische Maßnahme erforderlich, um eine Entwicklung in Richtung Z₁ zu erreichen. Von Zustand P aus können sich die Parameter durch die medizinische Maßnahme so verändern, daß sich der Zustand des Patienten in den ersten Raumbereich Z₁ oder den zweiten Raumbereich Z₂ verschiebt. Dies ist durch die Pfeile 6 und 7 angedeutet, die von dem Kreis P ausgehen. Die tatsächliche Entwicklungsrichtung kann der Behandler ändern, indem er einzelne Parameter so verändert, daß einer der Raumbereiche erreicht werden kann. Eine Entwicklung zu dem erwünschten Raumbereich Z₁ entlang Pfeil 6 kann somit frühzeitig beispielsweise durch Medikamentengabe erreicht werden.

Die Zieltrajektorie (Pfeil 1) kennzeichnet die erwünschte Entwicklung des Zustandes des Patienten von dem Ausgangszustand A zu dem erwünschen Zustand Z₁. Um diese Zieltrajektorie kann ein risikoabhängiger Toleranzbereich definiert werden. Die Weite des Toleranzbereichs ist abhängig von den individuellen Risikofaktoren wie z. B. dem vorhandenen Genotyp oder den aktivierten Onkogenen.

Vom Zustand A soll sich der Zustand des Patienten in Richtung des Raumbereiches Z₁ entwickeln (Pfeil 1 in Fig. 1). Dazu wird erfindungsgemäß wie folgt vorgegangen:
1. Der Patient wird am Ausgangszustand t₀ mit seinem vollständigen Parametersatz erfaßt.
2. Der Patient wird zum Zeitpunkt t₁ - tₙ in seinem vollständigen Parametersatz erfaßt. Die mehrfache Bestimmung des aktuellen Patientenzustandes verbessert die Möglichkeiten zur Bestimmung der Patiententrajektorie und ihre Lage zur Zieltrajektorie.
3. Zum Zeitpunkt t₁ können bereits der Abstand des aktuellen Patientenzustandes von der Zieltrajektorie und die Richtung, die die aktuelle Patiententrajektorie besitzt, berechnet werden.
4. Aus beiden Rechengrößen, dem Abstand und der Richtung der aktuellen Patiententrajektorie wird der kürzeste Weg in Richtung der Zieltrajektorie oder dem Raumbereich Z₁ berechnet.
5. Aus dem Abstand und der Richtung der Patiententrajektorie gegenüber der Zieltrajektorie. für den erwünschten Verlauf wird der kürzeste Weg berechnet und daraus die Therapievorschläge in Form des Handlungswertes abgeleitet.
6. Zur Berechnung der Trajektorien, des Abstandes zwischen Zieltrajektorie (Pfeil 1) und aktuellen Patientenzustand P sowie der Richtung der Patiententrajektorie (Pfeil 3) werden bekannte mathematische Verfahren, wie aus der analytischen Geometrie bekannt, eingesetzt.

Das erfindungsgemäß Verfahren ermöglicht somit die frühzeitige Erkennung von Fehlentwicklungen und gibt Hinweise, welche Parameter, die den Zustand des Patienten beschreiben, verändert werden können, um eine Entwicklung des Patienten hin zu einem erwünschten Zustand, dem ersten Raumbereich, zu erreichen. Beispielsweise kann mittels eines Computers der kürzeste Weg zum Erreichen des Zustandes Z₁, ausgehend von P, berechnet werden. Der Computer kann dazu Maßnahmen vorschlagen, wie einzelne oder mehrere Parameter verändert werden müssen.

### Beispiele

### Beispiel 1

In Beispiel 1 ist der Ausgangszustand A durch eine erfolgreiche kurative Mammakarzinom-Operation mit anschließender Bestrahlung und Chemotherapie gekennzeichnet. Der Merkmalsraum und die ersten und zweiten Raumbereiche Z₁, Z₂ werden anhand folgender Parameter gebildet: GOT, GPT, yGT, alkalische Phosphatase (AP), LDH, C-reaktives Protein (CRP), CEA, CA 15-3, Gluc, PTT, Hämatokrit, Zink, Selen, Zeit.

Als erwünschter Zustand (Raumbereich Z₁), zu dem sich der Zustand des Patienten, ausgehend vom Zustand A, entwickeln soll, ist eine Metastasenfreiheit über 20 Jahre bestimmt. Als unerwünschter Zustand (Raumbereich Z₂) wird ein Metastasennachweis innerhalb von 20 Jahren angesehen.

Die nachfolgende Tabelle 2 zeigt Werte für den Ausgangszustand A und den unerwünschten Zustand Z₂.

**Tabelle 2**

| Parameter | Ausgangszustand A | Zustand Z₂ |
|---|---|---|
| GOT | 12 U/l | 24 U/l |
| GPT | 15 U/l | 12 U/l |
| yGT | 4 U/l | 4 U/l |
| AP | 62 U/l | 62 U/l |
| LDH | 151 U/l | 295 U/l |
| CRP | 5 mg/l | 67 mg/l |
| CEA | 2 mg/l | 43 mg/l |
| CA 15-3 | 2 kU/l | 55 kU/l |
| Gluc | 5,2 mmol/l | 17,4 mmol/l |
| Hämatokrit | 46 1/1 | 59 1/1 |
| PTT | 35 s | 22 s |
| Zn | 1,3 mg/l | 0,26 mg/l |
| Se | 90 mg/l | 23 mg/l |

### Beispiel 2

In Beispiel 2 ist der Ausgangszustand A durch Beendigung einer Bypassoperation und Verlegung auf die Intensivstation mit Beatmung gekennzeichnet. Der Merkmalsraum und die ersten und zweiten Raumbereiche Z₁, Z₂ werden anhand folgender Parameter gebildet: pO₂, pCO₂, pH, AF, PEEP, CRP, PCT, Gluc, Selen, PTT, T, Zeit.

Als erwünschter Zustand (Raumbereich Z₁), zu dem sich der Zustand des Patienten, ausgehend vom Zustand A, entwickeln soll, sind Meßwerte für die ausgewählten Parameter definiert, die eine Verlegung von der Intensivstation. Als unerwünschter Zustand (Raumbereich Z₂) wird die Entwicklung einer beatmungsassoziierten Pneumonie angesehen.

Die nachfolgende Tabelle 3 zeigt Werte für den Ausgangszustand A und den unerwünschten Zustand Z₂.

**Tabelle 3**

| Parameter | Ausgangszustand A | Zustand Z₂ |
|---|---|---|
| pO₂ | 90 mmHg | 48 mmHg |
| pCO₂ | 40 mmHg | 51 mmHg |
| pH | 7,4 | 7,7 |
| AF | 15/min | 29/min |
| PEEP | 5 mbar | 22 mbar |
| CRP | 5 mg/l | 204 mg/l |
| PCT | 0,3 | 10 |
| Gluc | 4,8 mmol/l | 22,5 mmol/l |
| Se | 104 mg/l | 18 mg/l |
| PTT | 40 s | 18 s |
| T | 36,8 | 39,4 |

### Beispiel 3

In Beispiel 3 ist der Ausgangszustand A durch den Eintritt der Menopause gekennzeichnet. Der Merkmalsraum und die ersten und zweiten Raumbereiche Z₁, Z₂ werden anhand folgender Parameter gebildet: Ca⁺⁺, PO₃⁻⁻⁻ , AP, saure Phosphatase (SP), DH, PTH, Calcitonin (CT), Wachstumshormon STH, Osteocalcin, Vitamin D, Zeit.

Als erwünschter Zustand (Raumbereich Z₁), zu dem sich der Zustand des Patienten, ausgehend vom Zustand A, entwickeln soll, ist ein Knochenmasseverlust < 0,5 - 0,7 %/a (trabekulär) bzw. <- 0,5 - 0,6 %/a (kortikal) bestimmt. Als unerwünschter Zustand (Raumbereich Z₂) wird ein Knochenmasseverlust > 0,7 bzw. 0,6 %/a innerhalb der nächsten 5 Jahre angesehen.

Die nachfolgende Tabelle 4 zeigt Werte für den Ausgangszustand A und den unerwünschten Zustand Z₂.

**Tabelle 4**

| Parameter | Ausgangszustand | Zustand Z₂ |
|---|---|---|
| Ca⁺⁺ | 2,5 mmol/l | 2,4 mmol/l |
| PO₃⁻⁻⁻ | 1,2 mmol/l | 1,3 mmol/l |
| AP | 110 U/l | 200 U/l |
| SP | 10 U/l | 20 U/l |
| CT (Calcitonin) | 8 ng/l | 1 ng/l |
| STH (Wachstumshormon) | 4 m/l | nicht nachweisbar |
| Osteocalcin | 10 ng/l | 4 ng/l |
| Vitamin D | 125 pmol/l | 12 pmol/l |

Liste der verwendeten Bezugszeichen
- 1: Zieltrajektorie
- 2: Komplikationstrajektorie
- 3: Patiententrajektorie
- 4, 5: mögliche Entwicklungen der Patiententrajektorie vor Erreichen des Zustandes P
- 6, 7: mögliche Entwicklungen der Patiententrajektorie nach Erreichen des Zustand des P

- A: Ausgangszustand des Patienten (erster Meßpunkt)
- P: gegenwärtiger Zustand des Patienten
- Z₁: erster Raumbereich (erwünschter Zustand)
- Z₂: zweiter Raumbereich

## Patentansprüche

1. Verfahren zur Analyse und/oder Überwachung physiologischer und/oder pathologischer Parameter eines Patienten, umfassend
(a) das Auswählen physiologischer und/oder pathologischer Parameter, die für eine Erkrankung charakteristisch sind, wobei die Anzahl der ausgewählten Parameter mindestens 3 beträgt und wobei die ausgewählten physiologischen und/oder pathologischen Parameter mindestens zwei Parameter umfassen, die chemische Indikatorstoffe für das Vorliegen einer Erkrankung sind;
(b) das Bilden eines mehrdimensionalen Merkmalsraums, wobei jeder der ausgewählten Parameter eine Dimension des Merkmalsraums bildet und die Zeit eine weitere Dimension des Merkmalsraums bildet;
(c) das Bestimmen von zumindest einem ersten Raumbereich und von zumindest einem zweiten Raumbereich innerhalb des Merkmalsraums, wobei
im ersten Raumbereich jeder der ausgewählten Parameter und/oder die Zeit Werte aufweist, die als Zielwerte vorgegeben worden sind;
im zweiten Raumbereich zumindest einer der Parameter und/oder die Zeit Werte aufweist, die als unerwünschte Werte vorgegeben worden sind; und
(d) das Erfassen von Meßwerten des Patienten für die ausgewählten Parameter, die für einen ersten Zustand des Patienten charakteristisch sind, und das Einordnen der Meßwerte in den Merkmalsraum, wodurch ein erster Meßpunkt in dem Merkmalsraum gebildet wird;
**dadurch gekennzeichnet, daß** es ferner
(e) das Bestimmen der kürzesten Raumkurve zwischen dem ersten Meßpunkt und einem ersten Raumbereich mittels eines Computers unter Einsatz von mathematischen Verfahren der analytischen Geometrie und das Bestimmen des kürzesten Weges zwischen dem ersten Messpunkt und dem zumindest einen zweiten Raumbereich;
(f) das wiederholte Erfassen von Meßwerten für die ausgewählten Parameter und zeitabhängiges Einordnen dieser Meßwerte in den Merkmalsraum, wodurch weitere Meßpunkte in dem Merkmalsraum gebildet werden;
(g) das Erzeugen eines Handlungswertes mittels eines Computers, wenn einer der weiteren Meßpunkte außerhalb der Zieltrajektorie liegt, wobei der Handlungswert den oder die Parameter darstellt, die verändert werden müssen, um die Entwicklung der aus den Meßpunkten gebildeten Raumkurve (Patiententrajektorie) auf kürzestem Wege in Richtung des ersten Raumbereiches oder der Zieltrajektorie zu erreichen; und
(h) Übergeben des erzeugten Handlungswertes an ein Patientenwarnsystem, dass einen optischen oder akustischen Alarm auslöst;
umfaßt.

2. Verfahren nach Anspruch 1, **dadurch gekennzeichnet, daß** die in Schritt (a) ausgewählten physiologischen und/oder pathologischen Parameter Parameter umfassen, die für den Zustand des Gewebes, eines Organs oder des Organismus des Patienten charakteristisch sind und das Alter des Patienten und die Zusammensetzung des Gewebes umfassen.

3. Verfahren nach Anspruch 2, **dadurch gekennzeichnet, daß** die Zusammensetzung des Gewebes die stoffliche und/oder die zelluläre Zusammensetzung des Gewebes umfaßt.

4. Verfahren nach einem der vorstehenden Ansprüche, **dadurch gekennzeichnet, daß** die in Schritt (a) ausgewählten physiologischen und/oder pathologischen Parameter umfassen, die aus Gruppe ausgewählt sind, die aus
Parametern, die für akute oder frühere Entzündungen des Patienten charakteristisch sind;
Parametern, die für den Ernährungszustand des Patienten charakteristisch sind;
Parametern, die für akute oder frühere Infektionen des Patienten charakteristisch sind; und
Parametern, die für den physiologischen Alterungsprozeß charakteristisch sind;
besteht.

5. Verfahren nach Anspruch 4, **dadurch gekennzeichnet, daß** Parameter, die für den Ernährungszustand des Patienten charakteristisch sind, Parameter, die für den Wasser- und Elektrolytstoffwechsel des Patienten charakteristisch sind, und/oder Parameter umfassen, die für die Spurenelementversorgung charakteristisch sind.

6. Verfahren nach Anspruch 4, **dadurch gekennzeichnet, daß** Parameter, die für den physiologischen Alterungsprozeß charakteristisch sind, die Konzentration vorgegebener Hormone, Parameter, die für Veränderungen des Bindegewebsstoffwechsels charakteristisch sind, und mit Diabetes assoziierte Parameter umfassen.

7. Verfahren nach einem der vorstehenden Ansprüche, **dadurch gekennzeichnet, daß** die Bestimmung eines Handlungswertes in Schritt (g) das Bestimmen des kürzesten Weges zwischen dem zeitlich letzten Meßpunkt und einem ersten Raumbereich umfaßt.

8. Verfahren nach Anspruch 7, **dadurch gekennzeichnet, daß** der Handlungswert zumindest einen der ausgewählten Parameter angibt, der verändert werden soll, um den ersten Raumbereich auf den kürzesten Weg zu erreichen.

9. Verfahren nach einem der Ansprüche 1 bis 6, **dadurch gekennzeichnet, daß** die Bestimmung eines Handlungswertes in Schritt (g) das Bestimmen des kürzesten Weges zwischen dem zeitlich letzten Meßpunkt und der Zieltrajektorie umfaßt.

10. Verfahren nach Anspruch 9, **dadurch gekennzeichnet, daß** der Handlungswert zumindest einen Parameter angibt, der
verändert werden soll, um den ersten Raumbereich auf den kürzesten Weg zu erreichen.

11. Verfahren nach einem der vorstehenden Ansprüche, **dadurch gekennzeichnet, daß** der erste Meßpunkt und die in Schritt (f) bestimmten weiteren Meßpunkte zur Patiententrajektorie zusammengefaßt werden.

12. Verfahren nach einem der vorstehenden Ansprüche, **dadurch gekennzeichnet, daß** die Zieltrajektorie, die Patiententrajektorie und die Komplikationstrajektorie zur Bestimmung des Handlungswertes miteinander verglichen werden.

## Claims

1. A method for analyzing and/or monitoring physiological and/or pathological parameters of patient, comprising
(a) choosing physiological and/or pathological parameters typical for a disorder, wherein the number of chosen parameters is at least 3 and wherein the chosen physiological and/or pathological parameters comprise at least two parameters that are chemical indicators for the presence of a disease;
(b) forming a multidimensional feature space, wherein each of the chosen parameters forms a dimension of the feature space and the time forms a further dimension of the feature space;
(c) determining of at least a first space range and at least a second space range within the feature space, wherein
in the first space range each of the chosen parameters and/or the time have values that have been given as target values;
in the second space range at least one of the parameters and/or the time have values that have been given as unwanted values; and
(d) collecting measured values of the patient for the chosen parameters typical for a first condition of the patient and assigning of the measured values to the feature space, in order to form a first measured point in the feature space;
**characterized in that**, it further comprises
(e) determining of the shortest space curve between the first measured point and a first space range by means of a computer using mathematical methods of analytical geometry and determining the shortest way between the first measured point and the at least second space range;
(f) repeated collecting of measured values for the chosen parameters and time-dependent assigning of these measured values to the feature space, in order to form further measured points in the feature space;
(g) generating an instruction value by means of a computer, if one of the further measured points is outside of the target trajectory wherein the instruction value represents that or those parameter(s) that have to be changed in order to achieve in the shortest way the development of the space curve (patient trajectory) formed of the measured points towards the first space range or the target trajectory; and
(h) transferring the generated instruction value to a patient warning system triggering an optic or audible alarm.

2. The method according to claim 1, **characterized in that** the physiological and/or pathological parameters chosen in step (a) comprise parameters that are typical for the state of the tissue, an organ or of the organism of the patient and that comprise the age of the patient and the composition of the tissue.

3. The method according to claim 2, **characterized in that** the composition of the tissue comprises the material and/or cellular composition of the tissue.

4. The method according to anyone of the preceding claims, **characterized in that** the physiological and/or pathological parameters chosen in step (a) comprise parameters, selected from the group, consisting of
parameters typical for acute or earlier inflammations of the patient;
parameters typical for the nutritional state of the patient;
parameters typical for acute or earlier infections of the patient; and
parameters typical for the physiological aging process.

5. The method according to claim 4, **characterized in that** parameters typical for the nutritional state of the patient comprise parameters typical for the water and electrolyte metabolism of the patient and/or parameters typical for the trace element supply.

6. The method according to claim 4, **characterized in that** parameters typical for the physiological aging process comprise the concentration of given hormones, parameters typical for alterations of the connective tissue metabolism, and diabetes-associated parameters.

7. The method according to anyone of the preceding claims, **characterized in that** the determination of an instruction value in step (g) comprises the determination of the shortest way between the temporally last measured point and a first space range.

8. The method according to claim 7, **characterized in that** the instruction value indicates at least one of the chosen parameters to be changed to reach the first space range on the shortest way.

9. The method according to anyone of claims 1 to 6, **characterized in that** the determination of an instruction value in step (g) comprises the determination of the shortest way between the temporally last measured point and the target trajectory.

10. The method according to claim 9, **characterized in that** the instruction value indicates at least one parameter to be changed to reach the first space range on the shortest way.

11. The method according to anyone of the preceding claims, **characterized in that** the first measured point and the further measured points determined in step (f) are taken together for the patient trajectory.

12. The method according to anyone of the preceding claims, **characterized in that** the target trajectory, the patient trajectory, and the complication trajectory are compared with each other for determining the instruction value.

## Revendications

1. Procédé d'analyse et/ou de surveillance de paramètres physiologiques et/ou pathologiques d'un patient comprenant
(a) la sélection de paramètres physiologiques et/ou pathologiques caractéristiques pour une maladie, le nombre des paramètres sélectionnés étant au moins de 3 et les paramètres physiologiques et/ou pathologiques sélectionnés comprenant au moins deux paramètres qui sont des matières indicatrices chimiques pour la présence d'une maladie ;
(b) la formation d'un espace de caractéristiques multidimensionnel, chacun des paramètres sélectionnés formant une dimension de l'espace de caractéristiques et la durée formant une autre une dimension de l'espace de caractéristiques ;
(c) la détermination d'au moins une première zone d'espace et d'au moins une deuxième zone d'espace dans l'espace de caractéristiques,
dans la première zone d'espace de chacun des paramètres sélectionnés et/ou la durée présentant des valeurs attribuées comme valeurs-cible ;
dans la deuxième zone d'espace, au moins un des paramètres et/ou la durée présentant des valeurs attribuées comme valeurs non désirables ; et
(d) la saisie de valeurs mesurées sur le patient pour les paramètres sélectionnés caractéristiques pour un premier état du patient et le classement dans l'espace de caractéristiques des valeurs mesurées, ce qui forme un premier point de mesure dans l'espace de caractéristiques ;
**caractérisé en ce qu'**il comprend en plus
(e) la détermination de la courbe spatiale la plus courte entre le premier point de mesure et une première zone d'espace à l'aide d'un ordinateur en utilisant des procédés mathématiques de la géométrie analytique et la détermination du trajet le plus court entre le premier point de mesure et au moins de la deuxième zone d'espace ;
(f) la saisie répétée de valeurs mesurées pour les paramètres sélectionnés et un classement selon la durée de ces valeurs mesurées dans l'espace de caractéristiques, ce qui forme d'autres points de mesure dans l'espace de caractéristiques ;
(g) la génération d'une valeur d'action à l'aide d'un ordinateur l'un des autres points de mesure se trouve hors de la trajectoire cible; la valeur d'action représentant le ou les paramètres devant être modifié(s) pour atteindre le développement de la courbe spatiale formée à partir des points de mesure (trajectoire patient) sur le trajet le plus court en direction de la première zone d'espace ou de la trajectoire cible ; et
(h) transmission de la valeur d'action générée au système d'avertissement patient déclenchant une alarme optique ou acoustique.

2. Procédé selon la revendication 1 **caractérisé en ce que** les paramètres physiologiques et/ou pathologiques sélectionnés à l'étape (a) comprennent des paramètres étant caractéristiques de l'état du tissu, d'un organe ou de l'organisme du patient et comprenant l'âge du patient et la composition du tissu.

3. Procédé selon la revendication 2 **caractérisé en ce que** la composition du tissu comprend la composition matérielle et/ou cellulaire du tissu.

4. Procédé selon l'une des revendications précédentes **caractérisé en ce que** les paramètres physiologiques et/ou pathologiques sélectionnés à l'étape (a) comprennent des paramètres sélectionnés du groupe se composant de
paramètres caractéristiques pour des inflammations aiguës ou anciennes du patient; paramètres caractéristiques pour l'état de nutrition du patient ;
paramètres caractéristiques pour des infections aiguës ou anciennes du patient ; paramètres caractéristiques pour le processus de vieillissement physiologique.

5. Procédé selon la revendication 4 **caractérisé en ce que** les paramètres caractéristiques pour l'état de nutrition du patient comprennent les paramètres caractéristiques pour le métabolisme hydro-électrolytique du patient et/ou des paramètres caractéristiques pour l'approvisionnement en oligoéléments.

6. Procédé selon la revendication 4 **caractérisé en ce que** les paramètres caractéristiques pour le processus de vieillissement physiologique, la concentration d'hormones attribuées comprennent des paramètres caractéristiques pour les modifications du métabolisme du tissu conjonctif et des paramètres associés au diabète.

7. Procédé selon l'une des revendications précédentes **caractérisé en ce que** la détermination d'une valeur d'action dans l'étape (g) comprend la détermination du trajet le plus court entre le dernier point de mesure dans le temps et une première zone d'espace.

8. Procédé selon la revendication 7 **caractérisé en ce que** la valeur d'action indique au moins un des paramètres sélectionnés devant être modifié pour atteindre la première zone d'espace par le trajet le plus court.

9. Procédé selon l'une des revendications 1 à 6 **caractérisé en ce que** la détermination d'une valeur d'action à l'étape (g) comprend la détermination du trajet le plus court entre le dernier point de mesure dans le temps et la trajectoire cible.

10. Procédé selon la revendication 9 **caractérisé en ce que** la valeur d'action indique au moins un paramètre devant être modifié pour atteindre la première zone d'espace par le trajet le plus court.

11. Procédé selon l'une des revendications précédentes **caractérisé en ce que** le premier point de mesure et les autres points de mesure déterminés à l'étape (f) sont regroupés pour former la trajectoire patient.

12. Procédé selon l'une des revendications précédentes **caractérisé en ce que** la trajectoire cible, la trajectoire patient et la trajectoire de complication sont comparées entre elles pour déterminer la valeur d'action.
